# EUROPEAN PATENT SPECIFICATION

(11) **EP 1 339 745 B1**
(45) Date of publication and mention of the grant of the patent: **10.01.2007**
(21) Application number: 00978987.6
(22) Date of filing: 30.11.2000
(51) Int. Cl.: C07K 14/705, C07K 19/00, C12N 15/12, C12N 15/62, C12N 5/06, C12N 5/08, C12N 5/10, A61K 35/14, A61P 37/02

(54) **IMMUNOTHERAPEUTIC METHODS AND MOLECULES**
IMMUNTHERAPEUTISCHE METHODEN UND MOLEKÜLE
PROCEDES ET MOLECULES D'IMMUNOTHERAPIE

(43) Date of publication of application: 03.09.2003
(73) Proprietor: Imperial Innovations Limited, Imperial College London SW7 2AZ (GB)
(72) Inventor: STAUSS, Hans Josef, Imperial College Sch Medicine, London W12 0NN (GB); AMROLIA, Persis J., Imperial College Sch Medicine, London W12 0NN (GB)
(74) Representative: Miles, John Stephen
(86) International application number: PCT/GB2000/004566
(87) International publication number: WO 2002/044207

(56) References cited:
- WO-A-00/02922
- WO-A-92/13887
- US-A- 6 060 054
- WILSON DB ET AL.: "Immunogenicity. I. Use of Peptide Libraries to Identify Epitopes That Activate Clonotypic CD4+ T Cells and Induce T Cell Responses to Native Peptide Ligands" THE JOURNAL OF IMMUNOLOGY, vol. 163, 1999, pages 6424-6434, XP002187666
- MOTTEZ E ET AL.: "Cells Expressing a Major Histocompatibility Complex Class I Molecule with a Single Covalently Bound Peptide Are Highly Immunogenic" JOURNAL OF EXPERIMENTAL MEDICINE, vol. 181, February 1995 (1995-02), pages 493-502, XP000654243 cited in the application
- AMROLIA P.J. ET AL BLOOD vol. 96, no. 11, 16 November 2000, page 551A, XP001032882
- AMROLIA P.J. ET AL BLOOD vol. 101, 01 February 2003, pages 1007 - 1014

## Description

The present invention relates to immunotherapeutic methods, and molecules and cells for use in immunotherapeutic methods. In particular, the present invention relates to the immunotherapy of leukaemia.

There is evidence that anti-tumour cytotoxic T lymphocytes (CTL) play an important role *in vivo.* Tumour reactive CTL have been shown to mediate tumour regression in animal models (Kast *et al* (1989) *Cell* **59,** 603-614) and in man (Kawakami *et al* (1994) *Proc. Natl. Acad. Sci. USA* **91,** 6458-6462). As with all types of anti-tumour therapy, a problem that needs to be overcome is that the therapy must destroy or inactivate the target tumour cells to a useful extent but that the therapy must not destroy or inactivate non-tumour cells to a deleterious extent. In other words, it is desirable if the therapy is selective for tumour cells to a beneficial extent.

Much of the current work on immunotherapy of cancer makes use of the fact that certain tumours express polypeptides which are not expressed in the equivalent non-tumour tissue, or makes use of the fact that the tumour expresses a mutant form of a polypeptide which is not expressed in the non-tumour tissue. However, it is not always possible to identify polypeptides in a tumour which fall into this category, and so other target polypeptides which can form the basis of an immunotherapeutic approach have been identified.

Work in melanoma patients has shown that peptide-epitopes recognised by melanoma-reactive CTL are frequently derived from tissue-specific differentiation antigens. Recognition of differentiation antigens which are expressed in normal tissues seems to violate the rules of immunological tolerance; however, the CTL recognition of melanoma-associated differentiation antigens could be explained by the fact that they are normally only expressed in melanocytes which exist in relative small numbers at immunologically privileged sites, thus failing to establish tolerance. There is also evidence that prostate-specific differentiation antigens can serve as targets for CTL against tumours of the prostate.

WO 00/26249 shows that WT-1 and gata-1 can serve as targets for CTL against leukaemia.

US patent No. 6,060,054 to Staerz describes products for T-lymphocyte immno-suppression.

There remains, however, the need to develop new targets, and new methods of, immunotherapy.

Amrolia *et al* (2000, Blood 96, 11, 1, page 551a, Abstract 2365) discloses that allorestricted cytotoxic T cells specific for human CD45 show potent anti-leukemic activity.

CD45 is a haematopoietic differentiation antigen that is expressed on the surface of most haematopoietic cells. CD45 is an abundant, highly glycosylated surface protein and different isoforms of the protein are generated by alternative splicing of the CD45 gene. All haematopoietic malignancies express CD45 at similar levels as normal cells. Hence, CD45 is an unsuitable target for conventional immunotherapy in the autologous setting. However, we now show that it is an attractive target for immunotherapy of haematological malignancies after stem cell transplantation from HLA-mismatched donors. According to the invention, allo-restricted CTL specific for CD45 epitopes presented by recipient HLA molecules will selectively eliminate recipient haematopoietic cells (malignant and normal cells) but not donor haematopoietic cells. Alternatively, but without being bound by any theory, it is conceivable that recipient malignant haematopoietic cells are more susceptible to CTL killing than normal haematopoietic cells. In this case, the CTL may preferentially kill malignant haematopoietic cells although they express similar CD45 levels as normal haematopoietic cells.

Using the allo-MHC-restricted CTL approach, we have identified peptide epitopes in CD45 which may be presented by HLA-A0201 class I molecules and displayed on the surface of leukaemia cells expressing these proteins endogenously. HLA-A0201 negative responder individuals were used as a source of CTL specific for peptides presented by HLA-A0201 class I molecule, and this approach allows identification of HLA-A0201 presented peptides independent of possible tolerance of autologous CTL.

HLA-A0201 is the most common HLA-A haplotype.

For the avoidance of doubt, the terms HLA and MHC are used interchangeably in this specification.

To the inventor's knowledge, CD45 has not previously been considered for immunotherapy. In any event, the inventor has now shown that, surprisingly, the allorestricted approach is applicable though CD45 is expressed at similar levels in normal cells and cells with haematopoietic malignancies.

A first aspect of the invention provides a peptide comprising an HLA-binding peptide of human CD45 polypeptide or a variant of said peptide, wherein said peptide is as defined in Claim 1.

Preferably, the variant is one which binds an HLA molecule.

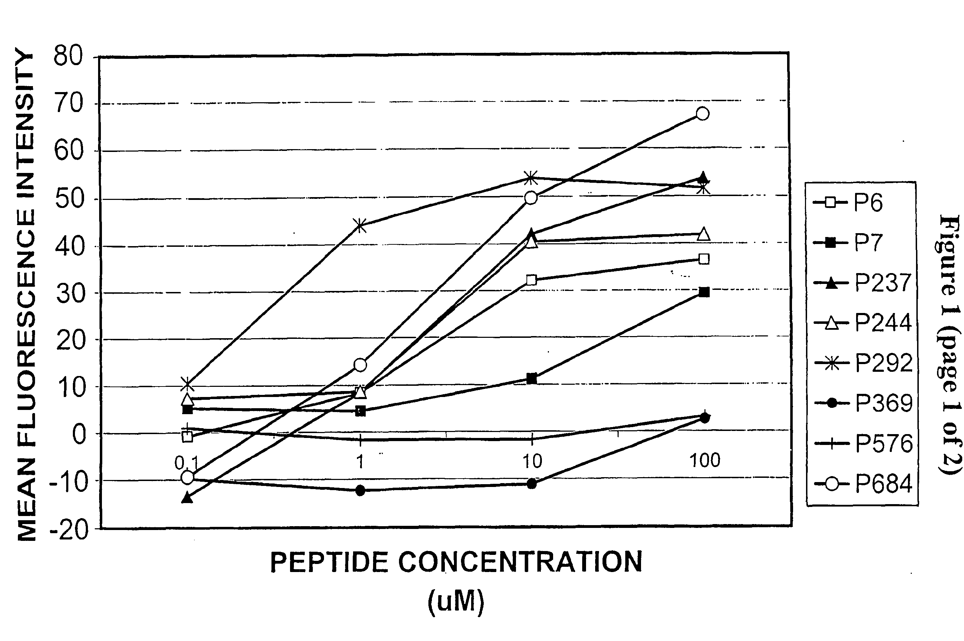

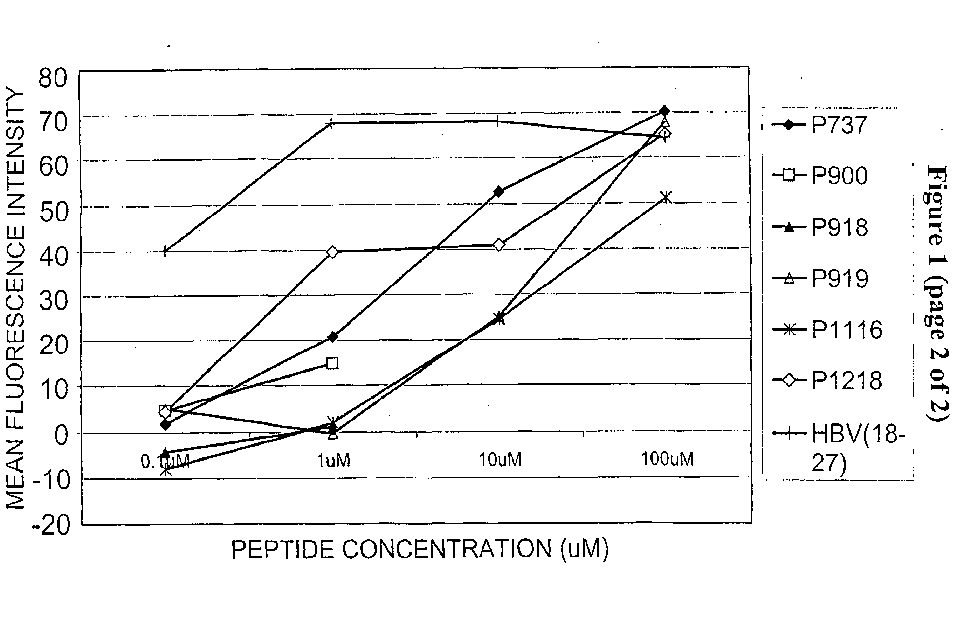

By "HLA-binding" peptide we mean a peptide which binds to one or more HLA molecules. Typically, a peptide will show HLA binding in a T2 binding assay when present in a concentration range of 10 µM to 1 nM. Whether or not a peptide of human CD45 is an HLA-binding peptide can be determined using methods known in the art. These methods include the T2 HLA stabilisation assay described in Figure 1, and the method described by Elvin *et al* (1993) *J. Immunol. Methods* **158**, 161-171.

By "peptide of human CD45 polypeptide" we mean a peptide which has contiguous amino acid sequence of the CD45 polypeptide. The peptide may be derived from the human CD45 polypeptide by chemical or enzymatic fragmentation, but it is preferred if the peptide is made by chemical synthesis or by expression using recombinant DNA technology as described below.

The sequence of CD45 is given in The Leukocyte Antigen Fact Book, 2nd edition, page 244, Academic Press, Harcourt, Brace & Co (publishers), 1997.

The peptide of the present invention consists of 9 to 12 amino acids and includes the amino acid sequence FLYDVIAST, or a variant of this sequence, which variant has one or two amino acid substitutions at either or both of positions 2 and 9 of this amino acid sequence, and which is capable of binding to HLA-A0201.

In an embodiment, the peptide consists of the amino acid sequence FLYDVIAST.

By "peptide" we include not only molecules in which amino acid residues are joined by peptide (-CO-NH-) linkages but also molecules in which the peptide bond is reversed. Such retro-inverso peptidomimetics may be made using methods known in the art, for example such as those described in Mézière *et al* (1997) *J. Immunol.* **159**, 3230-3237. This approach involves making pseudopeptides containing changes involving the backbone, and not the orientation of side chains. Mézière *et al* (1997) show that, at least for NHC class II and T helper cell responses, these pseudopeptides are useful. Retro-inverse peptides, which contain NH-CO bonds instead of CO-NH peptide bonds, are much more resistant to proteolysis.

It will be appreciated that the peptide may conveniently be blocked at its N-or C-terminus so as to help reduce susceptibility to exoproteolytic digestion.

By a "variant" of the given amino acid sequence we mean that the side chains of one or two of the amino acid residues are altered (for example by replacing them with the side chain of another naturally occurring amino acid residue or some other side chain) such that the peptide is still able to bind to an HLA molecule in substantially the same way as a peptide consisting of the given amino acid sequence. For example, a peptide may be modified so that it at least maintains, if not improves, the ability to interact with and bind a suitable MHC molecule, such as HLA-A0201, and so that it at least maintains, if not improves, the ability to generate activated CTL which can recognise and kill cells which express a polypeptide which contains an amino acid sequence as defined in the first aspect of the invention. (for example, CD45). Positions 2 and 9 of an HLA-A2-binding nonamer are typically anchor residues. Modifications of these residues involved in binding HLA-A2 may enhance binding without altering CTL recognition (for example, see Tourdot *et al* (1997) *J*. *Immunol.* **159**,2391-2398).

Thus, the peptides of the invention are ones which, typically, selectively and reversibly bind one or more HLA molecules; most typically they are able to bind HLA-A0201.

It will be appreciated from the following that in some applications the peptides of the invention may be used directly (ie they are not produced by expression of a polynucleotide in a patient's cell or in a cell given to a patient); in such applications the peptide has 12 or 11 or 10 or 9 residues.

It is preferred if the peptides of the invention are able to bind to HLA-A0201; however, the peptides may also bind to other HLA types as well as HLA-A0201. It is particularly preferred if the peptides bind selectively to HLA-A0201.

It is further preferred that the peptides of the invention are ones which can be used to generate peptide-specific CTL which show specific killing of T2 target cells when presenting the peptide (see Examples).

The peptides of the invention are particularly useful in immunotherapy, to target and kill cells which express the CD45 polypeptide.

Since these specific peptides consisting of the given amino acid sequences bind to HLA-A0201 it is preferred that the peptides of the invention are ones which bind HLA-A0201 and when so bound the HLA-A0201-peptide complex, when present on the surface of a suitable antigen-presenting cell, is capable of eliciting the production of a CTL which recognises a cell which expresses a polypeptide comprising the given amino acid sequence, such as the CD45 polypeptide.

It is well known that an optimum length for a peptide to bind to an HLA molecule is around 8 to 12 amino acid, preferably 9 amino acids.

A particular preferred peptide of the invention consists of the amino acid sequence FLYDVIAST.

Peptides (at least those containing peptide linkages between amino acid residues) may be synthesised by the Fmoc-polyamide mode of solid-phase peptide synthesis as disclosed by Lu *et al* (1981) *J. Org. Chem.* **46**, 3433 and references therein. Temporary N-amino group protection is afforded by the 9-fluorenylmethyloxycarbonyl (Fmoc) group. Repetitive cleavage of this highly base-labile protecting group is effected using 20% piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethylacrylamide polymer constituted from the three monomers dimethylacrylamide (backbone-monomer), bisacryloylethylene diamine (cross linker) and acryloylsarcosine methyl ester (functionalising agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexyl-carbodiimide/1-hydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50% scavenger mix. Scavengers commonly used are ethanedithiol, phenol, anisole and water, the exact choice depending on the constituent amino acids of the peptide being synthesised. Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from Calbiochem-Novabiochem (UK) Ltd, Nottingham NG7 2QJ, UK. Purification may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

The invention also includes a library of HLA-binding peptides of human CD45 polypeptide as defined in the first aspect of the invention wherein for each member of the library the type of HLA molecule it binds is recorded. The peptides may be stored in any suitable form in the library (eg frozen in solution or lyophilised) and the information concerning the type of HLA molecule bound by the peptide recorded in any suitable form, eg look-up table or computer record.

A second aspect of the invention provides a polynucleotide encoding a peptide as defined in the first aspect of the invention. The polynucleotide may be DNA or RNA and it may or may not contain introns so long as it codes for the peptide. Of course, it is only peptides which contain naturally occurring amino acid residues joined by naturally-occurring peptide bonds which are encodable by a polynucleotide.

A preferred polynucleotide of the invention is one which encodes an HLA heavy chain joined via a flexible linker to a peptide of the invention such that when the fusion molecule is expressed in a suitable cell the peptide binds the HLA peptide binding groove.

A third aspect of the invention provides an expression vector capable of expressing a polypeptide according to the first or aspect of the invention.

A variety of methods have been developed to operably link polynucleotides, especially DNA, to vectors for example *via* complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion as described earlier, is treated with bacteriophage T4 DNA polymerase or *E. coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Saiki *et al* (1988) *Science* **239**, 487-491. This method may be used for introducing the DNA into a suitable vector, for example by engineering in suitable restriction sites, or it may be used to modify the DNA in other useful ways as is known in the art.

In this method the DNA to be enzymatically amplified is flanked by two specific primers which themselves become incorporated into the amplified DNA. The said specific primers may contain restriction endonuclease recognition sites which can be used for cloning into expression vectors using methods known in the art.

The DNA (or in the case of retroviral vectors, RNA) is then expressed in a suitable host to produce a polypeptide comprising the compound of the invention (eg peptide according to the first aspect or fusion molecule according to the fourth aspect). Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention.

The DNA (or in the case of retroviral vectors, RNA) encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E*. *coli* and *Bacillus subtilis),* yeasts (for example *Saccharomyces cerevisiae),* filamentous fungi (for example *Aspergillus),* plant cells, animal cells and insect cells.

The vectors typically include a prokaryotic replicon, such as the ColE1 *ori,* for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E*. *coli,* transformed therewith. Suitable vectors are well known in the art.

The present invention also relates to a host cell transformed with a polynucleotide vector construct of the present invention. The host cell can be either prokaryotic or eukaryotic. Bacterial cells may be preferred prokaryotic host cells in some circumstances and typically are a strain of *E. coli* such as, for example, the *E*. *coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic and kidney cell lines. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650 and 293 cells which are human embryonic kidney cells. Preferred insect cells are Sf9 cells which can be transfected with baculovirus expression vectors. Transformation of host cells with DNA and vectors of the invention can be accomplished using methods known in the art.

It will be appreciated that certain host cells of the invention are useful in the preparation of the peptides or fusion molecules of the invention, for example bacterial, yeast and insect cells.

A fourth aspect of the invention provides a method of producing a peptide of the first aspect of the invention, the method comprising culturing host cells which contain a polynucleotide or expression vector which encodes the peptide and obtaining the peptide from the host cell or culture medium.

The peptides of the invention are used in the production of CTL specific for CD45 peptides and which are presented by particular HLA molecules, such as HLA-A0201.

Thus, further aspects of the invention provide compositions which comprise a peptide according to a first aspect of the invention which compositions are suitable for raising CTL specific for the peptides when presented by a particular HLA molecule. Such compositions are typically sterile and pyrogen free. Typically, for the generation of CTL the peptides are used in the range 200 µM to 1 nM.

Typically, the composition is an aqueous composition. In some instances it may be desirable to include a peptide-solubilising agent such as DMSO in the composition.

A still further aspect of the invention include a kit of parts comprising a peptide according to the first aspect of the invention and an antigen presenting cell. Preferred antigen presenting cells are described below. The kit of parts is useful in the preparation of activated CTL as described below. A yet still further aspect of the invention includes an antigen-presenting cell wherein its MHC Class I molecules are loaded with a peptide as defined in the first aspect of the invention. These cells are suitably comprised in a library of cells wherein each member cell of the library is loaded with a different peptide of the invention. As described in more detail below, it is convenient if for each peptide (and each cell within the library) an indication is given as to which type of Class I MHC molecule the peptide binds.

A further aspect of the invention provides a method for producing activated cytotoxic T lymphocytes (CTL) *in vitro,* the method comprising contacting *in vitro* CTL with antigen-loaded human class I MHC molecules expressed on the surface of a suitable antigen-presenting cell for a period of time sufficient to activate, in an antigen specific manner, said CTL wherein the antigen is a peptide according to the first aspect of the invention.

Suitably, the CTL are CD8⁺ cells but they may be CD4⁺ cells. The MHC class I molecules may be expressed on the surface of any, suitable cell and it is preferred if the cell is one which does not naturally express MHC class I molecules (in which case the cell is transfected to express such a molecule) or, if it does, it is defective in the antigen-processing or antigen-presenting pathways. In this way, it is possible for the cell expressing the MHC class I molecule to be loaded substantially completely with a chosen peptide antigen before activating the CTL.

The antigen-presenting cell (or stimulator cell) typically has an MHC class I molecule on its surface and preferably is substantially incapable of itself loading said MHC class I molecule with the selected antigen. As is described in more detail below, the MHC class I molecule may readily be loaded with the selected antigen *in vitro.*

Conveniently, said antigen-presenting cell is a mammalian cell defective in the expression of a peptide transporter such that, when at least part of said selected molecule is a peptide, it is not loaded into said MHC class I molecule.

Preferably the mammalian cell lacks or has a reduced level or has reduced function of the TAP peptide transporter. Suitable cells which lack the TAP peptide transporter include T2, RMA-S and *Drosophila* cells. TAP is the Transporter Associated with antigen Processing.

Thus, conveniently the cell is an insect cell such as a *Drosophila* cell.

The human peptide loading deficient cell line T2 is available from the American Type Culture Collection, 12301 Parklawn Drive, Rockville, Maryland 20852, USA under Catalogue No CRL 1992; the *Drosophila* cell-line Schneider line 2 is available from the ATCC under Catalogue No CRL 19863; the mouse RMA-S cell line is described in Karre and Ljunggren (1985) *J. Exp. Med.* **162,** 1745,

In a preferred embodiment the stimulator cell is a T2 cell or an RMA-S cell or a *Drosophila* cell transfected with a nucleic acid molecule capable of expressing said MHC class I molecule. Although T2 and RMA-S cells do express before transfection HLA class I molecules they are not loaded with a peptide.

Mammalian cells can be transfected by methods well known in the art. *Drosophila* cells can be transfected, as described in Jackson *et al* (1992) *proc. Natl. Acad. Sci. USA* **89**, 12117.

Conveniently said host cell before transfection expresses substantially no MHC class I molecules.

It is also preferred if the stimulator cell expresses a molecule important for T cell costimulation such as any of B7.1, B7.2, ICAM-1 and LFA 3.

The nucleic acid sequences of numerous MHC class I molecules, and of the costimulator molecules, are publicly available from the GenBank and EMBL databases.

It is particularly preferred if substantially all said MHC class I molecules expressed in the surface of said stimulator cell are of the same type.

HLA class I in humans, and equivalent systems in other animals, are genetically very complex. For example, there are more than 110 alleles of the HLA-B locus and more than 90 alleles of the HLA-A locus. Although any HLA class I (or equivalent) molecule is useful in this aspect of the invention, it is preferred if the stimulator cell presents at least part of the selected molecule in an HLA class I molecule which occurs at a reasonably high frequency in the human population. It is well known that the frequency of HLA class I alleles varies between different ethnic groupings such as Caucasian, African, Chinese and so on. At least as far as the Caucasian population is concerned it is preferred that HLA class I molecule is encoded by an HLA-A2 allele, or an HLA-A1 allele or an HLA-A3 allele or an HLA-B27 allele. HLA-A0201 is particularly preferred.

In a further embodiment, combinations of HLA molecules may also be used. For example, a combination of HLA-A2 and HLA-A3 covers 74% of the Caucasian population.

For therapeutic use of the CTL in the methods of the invention, allogeneic cells are used in the preparation of CTL and this method is described in detail in WO 97/26328. For example, in addition to *Drosophila* cells and T2 cells, other cells may be used to present antigens such as CHO cells, baculovirus-infected insects cells, bacteria, yeast, vaccinia-infected target cells. In addition plant viruses may be used (see, for example, Porta *et al* (1994) *Virology* **202**, 449-955 which describes the development of cowpea mosaic virus as a high-yielding system for the presentation of foreign peptides.

When allogeneic cells are used in the preparation of CTL, the CTL are allo-MHC-restricted with respect to the peptides of the invention. Thus, typically, the CTL are from an individual who is negative for a particular HLA and the peptide is presented by that particular HLA molecule by the antigen-presenting cell. It is particularly preferred that the CTL are from a HLA-A0201 negative responder individual and that the peptide is presented by a HLA-A0201 class I molecule by the antigen-presenting cell.

Exogenously applied peptides may be linked to a HIV tat peptide to direct them into the MHC Class I pathway for presentation by CTL (see, for example, Kim *et al* (1997) *J. Immunol.* **159**, 1666-1668).

The activated CTL which are directed against the peptides of the invention are useful in therapy. Thus, a further aspect of the invention provides activated CTL obtainable by the foregoing methods of the invention.

A still further aspect of the invention provides activated CTL which selectively recognise a cell which expresses a polypeptide as defined in the first aspect of the invention. Typically, the cell is a cell which expresses CD45. Preferably, the CTL recognises the said cell by binding to the peptide as defined in the first aspect of the invention. Thus, typically, the activated CTL recognises human Class I MHC molecules expressed on the surface of an antigen presenting cell and loaded with a peptide according to the first aspect of the invention.

The activated CTL of the invention may be packaged and presented for use in medicine. In particular, the HLA haplotype and the CD45 T cell epitope recognised by the CTL may be indicated. The invention also includes a pharmaceutical preparation comprising an activated CTL of the invention and a pharmaceutically acceptable carrier. Typically, the carrier is sterile and pyrogen free.

The CD45 polypeptide is expressed in leukaemias, such as lymphoid and myeloid leukaemias, and in other haematological malignancies. The CD45 molecule is also expressed in normal cells of the haematopoietic system. Thus, as discussed in more detail below, CD45 based immunotherapy will be combined with allogeneic stem cell transplantation. For example, the targeting of CD45 peptide epitopes presented by HLA-A0201 is provided for patients undergoing stem cell transplantation from HLA-A0201-negative donors. In this case, donor cells repopulate the patients haematopoietic system. These donor cells are resistant to CTL specific for HLA-A0201 presented peptide epitopes. Hence, such CTL selectively kill malignant and normal haematopoietic cells of the patient, but not normal haematopoietic cells of the stem cell donor. The allo-restricted approach is therefore used to generate CTL specific for CD45 peptides presented by HLA-A0201. As discussed in more detail below, the TCR of such allo-restricted CTL can be used for gene therapy of patients who might benefit from CD45-specific, HLA-restrieted CTL.

Thus, the CTL are suitable for killing target cells in a patient which target cells express a polypeptide comprising an ammo acid sequence as defined in the first aspect of the invention (eg CD45) wherein the patient is administered an effective number of the activated CTL. Typically, the patient has undergone stem cell transplantation from an HLA-mismatched donor and is administered an effective number of activated CTL derived from the same donor as the stem cell transplantation or from a further HLA-mismatched donor.

The target cells which are destroyed by the activated CTL are malignant and haematopoietic cells which express a polypeptide comprising an amino acid sequence given in the first aspect of the invention. Typically, the malignant and normal cells to be killed in the patient are haematopoietic cells which express CD45.

Hence, the CTL are not from the patient but are from another individual. Of course, it is preferred if the individual is a healthy individual. By "healthy individual" we mean that the individual is generally in good health, preferably has a competent immune system and, more preferably, is not suffering from any disease which can be readily tested for, and detected. In this embodiment, the CTL are derived from an individual whose HLA class I molecules are mismatched with those of the patient Thus, it is preferred if the CTL are allo-restricted. Treatment with allo-restricted CTL is described in WO 97/26328.

In order to readily select suitable CTL for treating a particular individual, the CTL may be held in a library. Thus, a further aspect of the invention provides a library of activated CTL wherein each member of the library (1) recognises a CD45 peptide when presented by a particular, recorded HLA and (2) has its HLA haplotype recorded.

The activated CTL may be stored in any suitable way, for example by cryopreservation in medium containing serum and DMSO as is well known in the art.

The information concerning the CD45 peptide recognised, and the HLA haplotype may be recorded in any suitable form eg in a look-up table or on computer. Preferably, each CTL is selective for a CD45 peptide.

Although not wishing to be bound by any theories, we believe that the use of allorestricted CTL directed against human CD45 relies on the principle that T-cell tolerance against epitopes from normally expressed proteins is self HLA-restricted. Thus, for example, an HLA-A2-negative donor may recognise epitopes presented by HLA-A2, to which an HLA-A2-positive host is tolerant CD45 is abundantly expressed in both malignant and normal cells of the haematopoietic lineage. Since it is expressed in a haematopoietic-specific fashion, we believe that CTL recognising CD45 should not cause toxicity to other host tissues (graft-versus-host disease): We believe that, by virtue of their allorestriction, such CTL should not kill the donor haematopoietic cells, which are HLA-A2 negative.

It is particularly preferred if the allorestricted CTL directed against human CD45 are administered to a patient who has previously had a haplo-identical transplantation for leukaemia (see *Ann. NYAcad. Sci.,* 1999, April 30, Vol 872 pp 351-361). Recent advances have made haplo-identical transplantation for leukaemia feasible. The allorestricted CTL approach discussed above is aimed at improving the graft-versus-leukaemia effect.

The CTL of the invention are suitable for adoptive immunotherapy.

The activated CTL contain a T cell receptor (TCR) which is involved in recognising cells which express the polypeptide. It is useful if the cDNA encoding the TCR is cloned from the activated CTL and transferred into a further CTL for expression.

The TCRs of CTL clones of the invention specific for the peptides of the first aspect of the invention are cloned. The TCR usage in the CTL clones is determined using (i) TCR variable region-specific monoclonal antibodies and (ii) RT-PCR with primers specific for Vα and Vβ gene families. A cDNA library is prepared from poly-A mRNA extracted from the CTL clones. Primers specific for the C-terminal portion of the TCR α and β chains and for the N-terminal portion of the identified Vα and β segments are used. The complete cDNA for the TCR α and β chain is amplified with a high fidelity DNA polymerase and the amplified products cloned into a suitable cloning vector. The cloned α and β chain genes may be assembled into a single chain TCR by the method as described by Chung *et al* (1994) *Proc. Natl. Acad. Sci. USA* **91**, 12654-12658. In this single chain construct the VαJ segment is followed by the VβDJ segment, followed by the Cβ segment followed by the transmembrane and cytoplasmic segment of the CD3 ζ chain. This single chain TCR is then inserted into a retroviral expression vector (a panel of vectors may be used based on their ability to infect mature human CD8⁺ T lymphocytes and to mediate gene expression: the retroviral vector system Kat is one preferred possibility (see Finer *et al* (1994) *Blood* **83**, 43). High titre amphotrophic retrovirus are used to infect purified CD8⁺ T lymphocytes isolated from the peripheral blood of tumour patients following a protocol published by Roberts *et al* (1994) Blood **84**, 2878-2889. Anti-CD3 antibodies are used to trigger proliferation of purified CD8⁺ T cells, which facilitates retroviral integration and stable expression of single chain TCRs. The efficiency of retroviral transduction is determined by staining of infected CD8⁺ T cells with antibodies specific for the single chain TCR. *In vitro* analysis of transduced CD8⁺ T cells establishes that they display the same tumour-specific killing as seen with the allo-resfiricted CTL clone from which the TCR chains were originally cloned. Populations of transduced CD8⁺ T cells with the expected specificity may be used for adoptive immunotherapy of the tumour patients. Patients may be treated with in between 10⁸ to 10¹¹ (most likely 10⁹-10¹⁰) autologous, transduced CTL.

Other suitable systems for introducing genes into CTL are described in Moritz *et al* (1994) *Proc. Natl. Acad. Sci. USA* **91**, 4318-4322. Eshhar *et al* (1993) *Proc. Natl. Acad. Sci. USA* **90**, 720-724 and Hwu *et al* (1993) *J. Exp. Med.* **178**, 361-366 also describe the transfection of CTL.

Thus, a further aspect of the invention provides a TCR which recognises a cell which expresses a polypeptide as defined in the first aspect of the invention, the TCR being obtainable from the activated CTL. Thus, typically, the TCR recognise a cell of the haematopoietic lineage which expresses CD45.

As well as the TCR, functionally equivalent molecules to the TCR are included in the invention. These include any molecule which is functionally equivalent to a TCR which can perform the same function as a TCR. In particular, such molecules include genetically engineered three-domain single-chain TCRs as made by the method described by Chung *et al* (1994) *Proc. Natl. Acad. Sci. USA* **91**, 12654-12658, and referred to above.

Typically, the TCR or a functionally equivalent molecule to the TCR, recognised human Class I MHC molecular expressed on the surface of an antigen-presenting cell and loaded with a peptide according to the first aspect of the invention.

The invention also includes a polynucleotide encoding the TCR or functionally equivalent molecule, and an expression vector encoding the TCR or functionally equivalent molecule thereof. Expression vectors which are suitable for expressing the TCR of the invention include viral vectors such as retroviral vectors, lentiviral vectors, adenoviral vectors, vaccinia vectors (including the replication-deficient MVA strain). It is, however, preferred that the expression vectors are ones which are able to express the TCR in a CTL following transfection.

A still further aspect of the invention provides the use of CTL in the manufacture of a medicament for a killing target cells in a patient, which target cells express a polypeptide comprising an amino acid sequence given in the first aspect of the invention, wherein the CTL are produced by a method comprising the steps of (1) obtaining CTL from the patient; (2) introducing into said cells a polynucleotide encoding a TCR, or a functionally equivalent molecule, as defined above. Typically, the target cells are malignant haematopoietic cells which express CD45.

It will be appreciated that, with respect to the methods of killing target cells in a patient, it is particularly preferred that the target cells are leukaemia cells or other malignant haematopoietic cells which express CD45.

The CD45 polypeptide comprises the amino acid sequences FLYDVIAST and ALLAFLAFL and KLFTAKLNV and MIWEQKATV and NLSELHPYL and VNLSELHPYL and LLAFGFAFL and YLYNKETKL and TLILDVPPGV and ILYNNHKFT and ILPYDYNRV and YILIHQALV and KLLAFGFAFL and YQYQYTNWSV.

It is particularly preferred if the patients who are treated by the methods of the invention have the HLA-A0201 haplotype. Thus, in a preferred embodiment the HLA haplotype of the patient is determined prior to treatment. HLA haplotyping may be carried out using any suitable method; such methods are well known in the art.

As discussed in detail above, allorestricted CTLs are used. Thus, it is convenient to determine the HLA haplotype of the donor and to use HLA-mismatched donor cells (at least mismatched for the HLA Class which presents the antigen). Thus, for example, if the patient is typed as HLA-A0201-positive, it is preferred that the donor for the CTL is HLA-A0201-negative so that the CTL are allorestricted.

Related methods, which as medical methods are not part of the invention, include a method of treating a patient with a haematopoietic malignancy, the method comprising (1) determining for a given HLA-binding peptide of human CD45 which type of Class I MHC molecule binds the peptide in the patient, or determining for a given Class I MHC molecule of the patient which peptide (or peptides) of human CD45 binds the Class I MHC molecule in the patient, or both, (2) providing an activated CTL which is allogeneic (allorestricted) with respect to the Class I MHC molecule which binds the peptide in the patient and which CTL is specific for the peptide, (3) undertaking a stem cell transplantation of the patient from a donor who is negative for the type of Class I MHC molecule which, in the patient, binds the peptide, and (4) administering the activated CTL of step (2) to the patient

The invention provides the use of activated CTL in the manufacture of a medicament for treating a patient with a haematopoietic malignancy, as defined in claim 32.

Preferably, the peptide of CD45 which binds the MHC Class I molecule is one which may be produced by natural processing of the CD45 antigen.

Typically, the haematopoietic malignancy is leukaemia.

The determination of which type of Class I MHC molecule binds a particular CD45 HLA-binding peptide may be determined empirically for the patient in question using well known methods. Alternatively, a look-up table for which HLA molecules bind which particular CD45 peptides may readily be generated, for example, Example 1 shows a list of peptides which bind HLA-A0201. In that case, which type of Class I MHC molecule binds the particular peptide can be determined simply by determining whether the patient has the type of Class I MHC molecule to which the particular peptide is known to bind. Determining the type of Class I molecule can be by DNA analysis as is well known in the art. By way of example, the peptide FLYDVIAST binds HLA-A0201 and for this peptide if a patient is shown to have an HLA-A0201 allele the patient is expected to bind this peptide with this MHC molecule.

The allogeneic (allorestricted) activated CTL may be produced using the method described above. The activated CTL may conveniently be ones which are known to be peptide specific and for which the HLA genotype has been determined. Thus, the CTL may be selected from a library of CTL which are classified with respect to their peptide specificity and HLA genotype.

The activated CTL are believed to be able to destroy any residual malignant haematopoietic cells (and also any of the patient's original normal haematopoietic cells) but not the transplanted cells.

The invention includes in particular the use of the peptides of the invention (or polynucleotides encoding them) and to activate CTL from healthy donors (MHC mismatched) *in vitro* followed by adoptive therapy. The invention will now be described in more detail by reference to the following Figures and Examples in which:

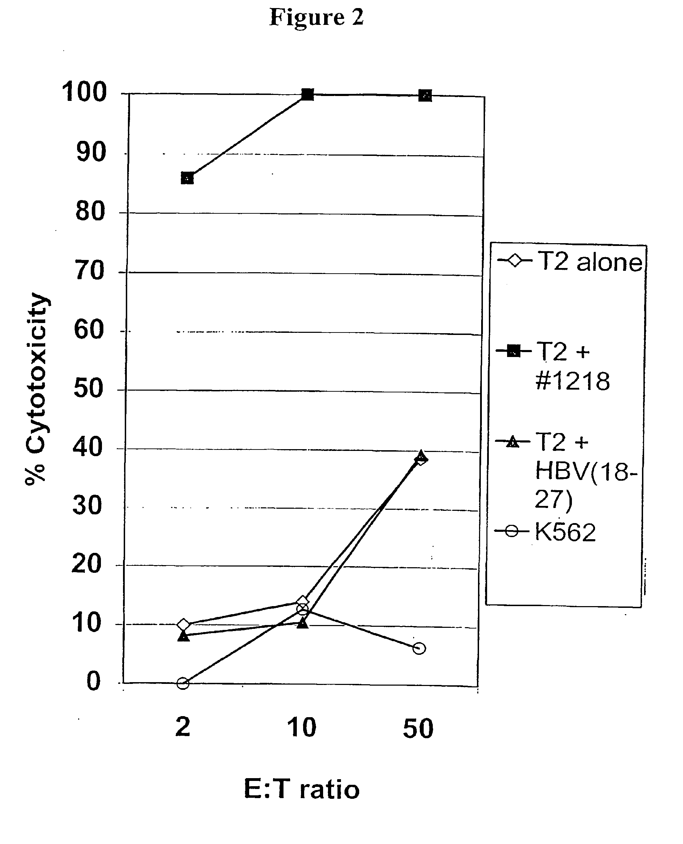

Figure 1 shows the result of an assay to measure HLA-A0201 binding of peptides. Peptide binding was measured using the T2 assay that is based on the ability of peptides to stabilise HLA-A0201 expression in the TAP-deficient T2 cells. The peptide 1218 (FLYDVIAST) was one of the best binders and was used to stimulate CTL responses (see Figure 2).
Figure 2 shows the specificity of 3 CTL lines generated against the CD45-derived peptide 1218. Peptide-specific CTL were isolated from HLA-A0201 negative donors using the method described in WO 97/26328. Three peptide-specific lines were established and found to show specific killing of T2 target cells presenting peptide 1218 but not targets presenting control HLA-A0201-binding peptides (HBV(18-27)). The NK target cells K562 were not killed by these CTL.

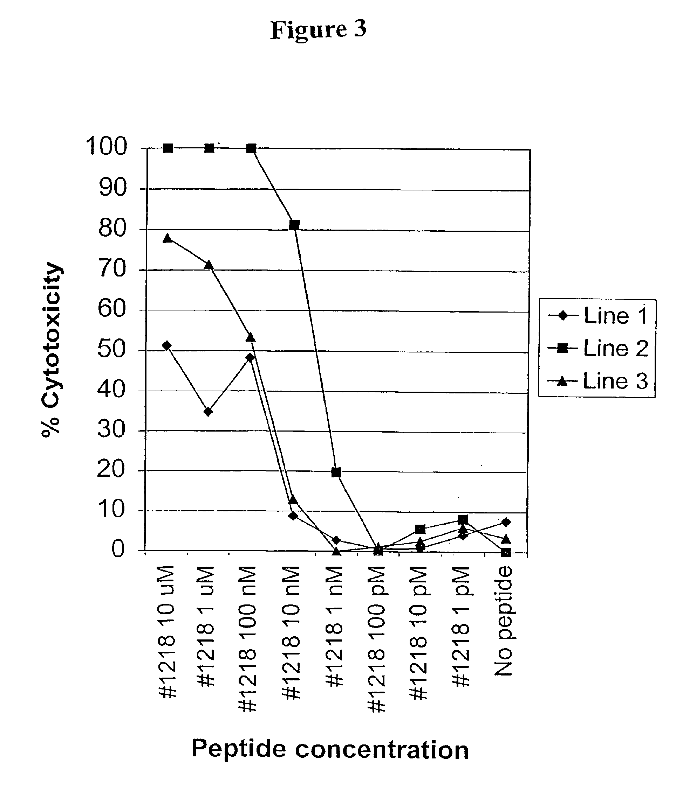

Figure 3 shows the sensitivity of 3 CTL lines generated against the CD45-derived peptide 1218. The sensitivity of the three CTL lines was tested using T2 target cells coated with decreasing doses of peptide 1218. CTL line 2 was the most sensitive line and used in subsequent experiments.

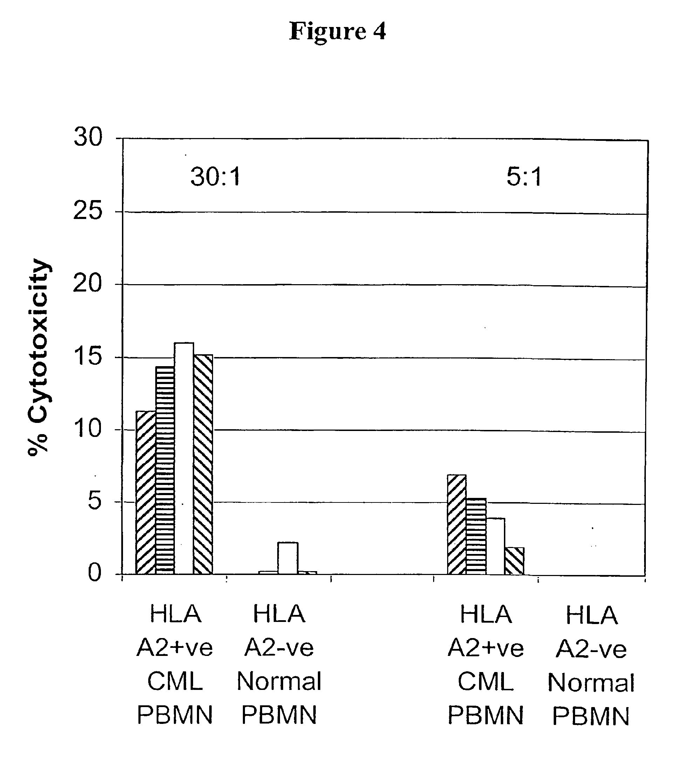

Figure 4 shows the killing of fresh peripheral blood mononuclear cells (PBMC) from CML (chronic myeloid leukaemia) patients by CTL specific for peptide 1218. CTL line 2 was used to test the killing activity against fresh PBMC from HLA-A0201 positive CML patients. The CTL were also tested against PBMC from HLA-A0201-negative individuals. Effector to target ratio was 30:1 and 5:1.

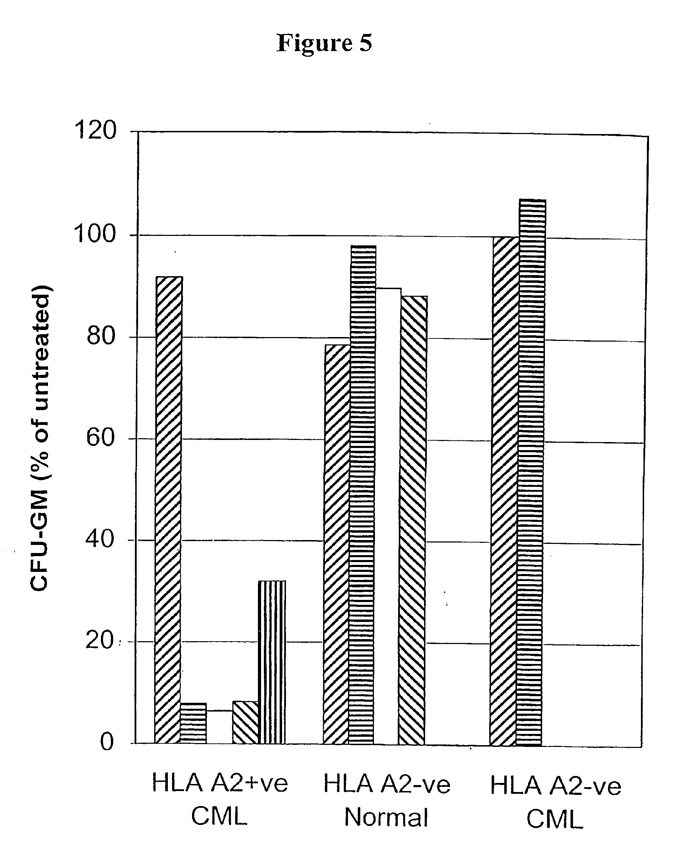

Figure. 5 shows the killing of clonogenic progenitor cells by CTL specific for peptide 1218. CTL line 2 was tested in colony forming assays. The CTL killed most of the colony forming progenitors from HLA-A0201 positive CML patients but not from HLA-A0201 negative normal individuals or CML patients. Purified CD34 progenitor cells were exposed to CTL for 4 hours followed by plating of the treated and control cells to measure the CFU-GM numbers. Shown is the number of colonies in treated CD34 cells relative to the number of colonies in untreated CD34 cells. Treatment eliminated most colonies in CD34 cells of HLA-A0201 positive CML patients.

### Example 1: HLA-A0201-binding peptides from human CD45

Sixteen peptides of the CD45 molecule were selected and 14 of them we tested in HLA-A0201 binding assays as described in the legend to Figure 1. Twelve of the peptides showed binding activity (see Figure 1).

**CD45 peptides**

| | |
|---|---|
| huCD45/1218 | FLYDVIAST |
| huCD45/576 | ALIAFLAFL* |
| huCD45/244 | KLFTAKLNV* |
| huCD45/737 | MIWEQKATV* |
| huCD45/919 | NLSELHPYL* |
| huCD45/918 | VNLSELHPYL* |
| huCD45/7 | LLAFGFAFL * |
| huCD45/237 | YLYNKETKL * |
| huCD45/293 | LILDVPPGV * |
| huCD45/292 | TLILDVPPGV* |
| huCD45/369 | ILYNNHKFT* |
| huCD45/684 | ILPYDYNRV* |
| huCD45/900 | YILIHQALV* |
| huCD45/304 | FQLHDCTQV* |
| huCD45/6 | KLLAFGFAFL* |
| huCD45/1116 | YQYQYTNWSV* |

| | |
|---|---|
| The underlined peptides (huCD45/293 and huCD45/304) were not tested for binding activity. these peptides are cited for illustrative purposes only. | |

### Example 2: Stimulation of CTL responses by peptide 1218

The Peptide 1218 was one of the best binders and was used to stimulate CTL responses. Peptide-specific CTL were isolated from HLA-A0201 negative donors using the method described in WO 97/26328. Three peptide-specific lines were established and found to show specific killing of T2 target cells presenting peptide 1218 but not targets presenting control peptides. The NK target cells K562 were not killed by these CTL (Figure 2).

The sensitivity of the three CTL lines was tested using T2 target cells coated with decreasing doses of peptide 1218. CTL line 2 was the most sensitive line (Figure 3).

CTL line 2 was used to test the killing activity against fresh leukaemic cells from HLA-A0201 positive CML patients. The CTL showed killing of these CML cells but not of normal cells from HLA-A0201-negative donors (Figure 4).

Finally, CTL line 2 was tested in colony forming assays. The CTL killed most of the colony forming progenitors from HLA-A0201+ CML patients but not from HLA-A0201- individuals (Figure 5).

These data show that CD45 has at least 12 peptides that can bind to HLA-A0201. Peptide 1218 can induce peptide specific CTL from HLA-A0201 negative donors. The CTL which show 1218 peptide specificity using T2 target cells also kill HLA-A0201 positive CML cells.

We have screened 14 candidate peptides from CD45 for binding to HLA-A201 in T2 binding assays. Using one of the best binding peptides (p1218), we can reliably generate peptide-specific, allorestricted CTL. Three p1218 specific CTL lines show potent cytotoxicity against HLA A2+ve (C1RA2) but not A2 -ve (WS29 or K562) hematopoietic cell lines, although some allreactivity against A2+ve, non-hematopoietic targets was observed. p1218 line 2, which had the highest avidity in peptide tritration assays, showed significant cytotoxicity against PGMN in 4/5HLA A2+ve patients with CML, including 1 in myeloid blast crisis, but 0/4 HLA A2-ve normal controls. Treatment of CD34+ve PBMN/BMMN with p1218 specific CTL inhibited CFU-GM colony formation by >90% in 4/5 HLA A2+ve CML patients without significant inhibition in 5/5 HLA A2-ve normal controls, demonstrating that p1218-specific CTL have potent activity against leukemic progenitors. These data suggest that adoptive immunotherapy with allorestricted CTL directed against CD45 epitopes may be useful in restoring the graft-versus-leukemia effect post haplo-identical transplantation.

### Example 3: Production of activated cytotoxic lymphocytes (CTL) using Class I molecules and the CD45 peptide antigen FLYDVIAST and their administration

Activated cytotoxic T lymphocytes (CTLs) are produced using HLA-A2 Class I molecules and the nonamer peptide from CD45: FLYDVIAST.

The method described in PCT patent application WO 93/17095 is used to make the CTLs. Cells with an endogenous defect in the peptide loading of HLA Class I molecules are used as stimulator cells. For example, human T2 cells, murine RMA-S/A2 cells or *Drosophila* cells transfected with human HLA-A0201, B7.1 and ICAM-1. The HLA-A0201 molecules expressed in these cells can be conveniently loaded with exogenously supplied peptides.

The peptide is synthesised on an Applied Biosystems synthesiser, ABI 431A (Foster City, CA, USA) and subsequently purified by HPLC.

As is described in detail in WO 93/17095, in order to optimize the *in vitro* conditions for the generation of specific cytotoxic T cells, the culture of stimulator cells is maintained in an appropriate medium. The stimulator cells are T2 or RMA-S/A2 or *Drosophila* cells as described in WO 93/17095.

Prior to incubation of the stimulator cells with the cells to be activated, eg precursor CD8 cells, an amount of antigenic peptide is added to the stimulator cell culture, of sufficient quantity to become loaded onto the human Class I molecules to be expressed on the surface of the stimulator cells. A sufficient amount of peptide is an amount that will allow about 200, and preferably 200 or more, human Class I MHC molecules loaded with peptide to be expressed on the surface of each stimulator cell. The stimulator cells are typically incubated with >1 µg/ml peptide.

Resting or precursor CD8 cells are then incubated in culture with the appropriate stimulator cells for a time period sufficient to activate the CD8 cells. The CD8 cells shall thus be activated in an antigen-specific manner. The ratio of resting or precursor CD8 (effector) cells to stimulator cells may vary from individual to individual and may further depend upon variables such as the amenability of an individual's lymphocytes to culturing conditions. The lymphocyte:stimulator cell *(Drosophila* cell) ratio is typically in the range of about 2:1 to 100:1. For example, 3 x 10⁷ human PBL and 3 x 10⁶ live *Drosophila* cells are admixed and maintained in 20 ml of RPMI 1640 culture medium.

The effector/stimulator culture are maintained for as long a time as is necessary to stimulate a therapeutically usable or effective number of CD8 cells. This usually requires several rounds of bulk stimulation followed by limiting dilution cultures to isolate peptide-specific CTL lines.

Effective, cytotoxic amounts of the activated CD8 cells can vary between *in vitro* and *in vivo* uses, as well as with the amount and type of cells that are the ultimate target of these killer cells between about 1 x 10⁶ and 1 x 10¹² activated CTL are used for adult humans.

Methods of re-introducing cellular components are used such as those exemplified in US Patent No 4,844,893 to Honsik *et al* and US Patent No 4,690,915 to Rosenberg. For example, administration of activated CD8 cells via intravenous infusion is appropriate.

## Claims

1. A peptide consisting of from 9 to 12 amino acids and including the amino acid sequence FLYDVIAST or a variant of this sequence, which variant has one or two amino acid substitutions at either or both of positions 2 and 9 of this amino acid sequence, and which is capable of binding to HLA-A0201.

2. A peptide according to Claim 1 wherein when bound to HLA-A0201 the peptide-bound HLA-A0201 is capable of eliciting the production of a cytotoxic T lymphocyte (CTL) which recognises a cell which expresses a polypeptide comprising the given amino acid sequence.

3. A peptide according to Claims 1 or 2 wherein the peptide includes non-peptide bonds.

4. A peptide according to Claim 1 consisting of the amino acid sequence FLYDVIAST.

5. A polynucleotide encoding a peptide according to any one of Claims 1, 2 and 4.

6. A polynucleotide according to Claim 5 which is DNA.

7. An expression vector capable of expressing a peptide according to any one of Claims 1, 2 and 4.

8. A host cell comprising a polynucleotide according to Claim 5 or 6 or an expression vector according to Claim 7.

9. A method of producing a peptide according to any one of Claims 1, 2 and 4 the method comprising culturing the host cell according to Claim 8 and obtaining the peptide from the host cell or its culture medium.

10. A kit of parts comprising a peptide according to any one of Claims 1 to 4 and an antigen presenting cell.

11. A kit of parts according to Claim 10 wherein the antigen presenting cell is a cell defective in, or lacks, the expression of the TAP peptide transporter.

12. A kit of parts according to Claim 11 wherein the cell is any one of a T2 cell, an RMA-S cell or a *Drosophila* cell.

13. An antigen-presenting cell wherein its MHC Class I molecules are loaded with a peptide according to any one of Claims 1 to 4.

14. A cell according to Claim 13 which is defective in, or lacks, the expression of the TAP peptide transporter.

15. A cell according to Claim 14 which is any of a T2 cell, an RMA-S cell or a *Drosophila* cell.

16. A method for producing activated cytotoxic T lymphocytes (CTL) *in vitro,* the method comprising contacting *in vitro* CTL with antigen-loaded human class I MHC molecules expressed on the surface of a suitable antigen-presenting cell for a period of time sufficient to activate, in an antigen specific manner, said CTL
wherein the antigen is a peptide according to any one of Claims 1 to 4.

17. A method according to Claim 16 wherein the CTL and the antigen-presenting cell are allogeneic with respect to the class I MHC molecule that is presenting the peptide according to any of Claim 1 to 4.

18. A method according to Claim 16 or 17 wherein the antigen is loaded onto class I MHC molecules expressed on the surface of a suitable antigen-presenting cell by contacting a sufficient amount of the antigen with an antigen-presenting cell wherein before contact the class I MHC molecules of the antigen-presenting cell are substantially unoccupied and after contact the class I MHC molecules are substantially fully occupied.

19. A method according to Claim 16 or 17 wherein the antigen-presenting cell comprises an expression vector which expresses a peptide according to Claim 7.

20. A method according to any one of Claims 16 to 18 wherein the class I MHC molecule is HLA-A0201.

21. Activated cytotoxic T lymphocytes (CTL) obtainable by the method according to any one of Claims 16 to 20, which selectively recognise a cell which expresses a polypeptide comprising an amino acid sequence given in Claim 1.

22. Activated cytotoxic T lymphocytes (CTL) according to Claim 21 which selectively recognise a malignant cell which expresses CD45.

23. A T-cell receptor (TCR) which recognises a cell which expresses a polypeptide comprising an amino acid sequence given in Claim 1, the TCR being obtainable from the cytotoxic T lymphocyte (CTL) of Claim 21 or 22, or a functionally equivalent molecule to the TCR.

24. A T-cell receptor (TCR) according to claim 23 cell which recognises a malignant haematopoietic cell which expresses CD45.

25. A polynucleotide encoding a T cell receptor (TCR) as defined in Claims 23 or 24.

26. An expression vector capable of expressing a T cell receptor (TCR) as defined in Claims 23 or 24.

27. Use of cytotoxic T lymphocytes as defined in Claim 21 or 22 in the manufacture of a medicament for killing target cells in a patient which target cells express a polypeptide comprising an amino acid sequence given in Claim 1.

28. Use of cytotoxic T lymphocytes in the manufacture of a medicament for killing target cells in a patient which target cells express a polypeptide comprising an amino acid sequence given in Claim 1, wherein the cytotoxic T lymphocytes are produced by a method comprising the steps of (1) obtaining cytotoxic T lymphocytes (CTL) from the patient; and (2) introducing into said cells a polynucleotide encoding a T cell receptor (TCR), or a functionally equivalent molecule, as defined in Claims 23 or 24.

29. A use according to Claim 27 wherein the patient has undergone an allogeneic stem cell transplantation.

30. A use according to any one of Claims 27 to 29 wherein the target cells are cancer cells.

31. A use according to Claim 30 wherein the cancer is a leukaemia which expresses the CD45 polypeptide.

32. Use of an activated CTL in the manufacture of a medicament for treating a patient with a haematopoietic malignancy, wherein the activated CTL is specific for an HLA-binding peptide of human CD45 as defined in claim 1, which peptide is bound by an HLA-A0201 molecule in the patient, and which activated CTL is allogeneic (allorestricted) with respect to HLA-A0201, and wherein the patient receives a stem cell transplantation from a donor who is HLA-A0201 negative, wherein the stem cell is not a human embryonic cell.

33. Use of stem cells from a donor who is HLA-A0201-negative in the manufacture of a medicament for treating a patient with a haematopoietic malignancy, wherein the patient is administered an activated CTL that is specific for an HLA-binding peptide of human CD45 as defined in claim 1, which peptide is bound by an HLA-A0201 molecule in the patient, and wherein the activated CTL is allogeneic (allorestricted) with respect to HLA-A0201, wherein the stem cell is not a human embryonic cell.

34. A use according to Claim 32 or 33 wherein the activated CTL are produced by the method of Claim 16 wherein the CTL are allogeneic (allorestricted) with respect to HLA-A0201.

35. A use according to Claim 32 or 33 wherein the activated CTL are selected from a library of CTL.

36. A library of activated CTL wherein each member of the library (1) recognises a peptide as defined in claim 1 when presented by a particular, recorded HLA and (2) has its HLA haplotype recorded.

37. A library of peptides as defined by Claim 1 wherein for each member of the library the type of HLA molecule it binds is recorded.

## Patentansprüche

1. Peptid, das aus 9 bis 12 Aminosäuren besteht und die Aminosäuresequenz FLYDVIAST oder eine Variante dieser Sequenz einschließt, wobei die Variante eine oder zwei Aminosäuresubstitution(en) in einer oder beiden der Positionen 2 und 9 dieser Aminosäuresequenz aufweist, und das zur Bindung an HLA-A0201 fähig ist.

2. Peptid gemäß Anspruch 1, wobei, wenn es an HLA-A0201 gebunden ist, das peptidgebundene HLA-A0201 in der Lage ist, die Produktion eines zytotoxischen T-Lymphozyten (CTL) auszulösen, der eine Zelle erkennt, die ein Polypeptid exprimiert, das die angegebene Aminosäuresequenz aufweist.

3. Peptid gemäß den Ansprüchen 1 und 2, wobei das Peptid Bindungen, die keine Peptidbindungen sind, enthält.

4. Peptid gemäß Anspruch 1, das aus der Aminosäuresequenz FLYDVIAST besteht.

5. Polynucleotid, das für ein Peptid gemäß einem beliebigen der Ansprüche 1, 2 und 4 codiert.

6. Polynucleotid gemäß Anspruch 5, bei dem es sich um DNA handelt.

7. Expressionsvektor, der in der Lage ist, ein Peptid gemäß einem beliebigen der Ansprüche 1, 2 und 4 zu exprimieren.

8. Wirtszelle, die ein Polynucleotid gemäß Anspruch 5 oder 6 oder einen Expressionsvektor gemäß Anspruch 7 aufweist.

9. Verfahren zur Erzeugung eines Peptids gemäß einem beliebigen der Ansprüche 1, 2 und 4, wobei das Verfahren das Kultivieren der Wirtszelle gemäß Anspruch 8 und das Gewinnen des Peptids aus der Wirtszelle oder deren Kulturmedium umfasst.

10. Kit aus Teilen, der ein Peptid gemäß einem beliebigen der Ansprüche 1 bis 4 und eine antigenpräsentierende Zelle umfasst.

11. Kit aus Teilen gemäß Anspruch 10, wobei die antigenpräsentierende Zelle eine Zelle ist, die bezüglich der Expression des TAP-Peptid-Transporters defekt ist oder diesen nicht exprimiert.

12. Kit aus Teilen gemäß Anspruch 11, wobei die Zelle eine beliebige von einer T2-Zelle, einer RMA-S-Zelle oder einer *Drosophila-*Zelle ist.

13. Antigenpräsentierende Zelle, wobei ihre MHC-Moleküle der Klasse I mit einem Peptid gemäß einem beliebigen der Ansprüche 1 bis 4 beladen sind.

14. Zelle gemäß Anspruch 13, die bezüglich der Expression des TAP-Peptid-Transporters defekt ist oder diesen nicht exprimiert.

15. Zelle gemäß Anspruch 14, die eine beliebige von einer T2-Zelle, einer RMA-S-Zelle oder einer *Drosophila-*Zelle ist.

16. Verfahren zur Erzeugung aktivierter zytotoxischer T-Lymphozyten (CTL) *in vitro,* wobei das Verfahren umfasst das In-Kontakt-bringen *in vitro* von CTL mit den antigenbeladenen humanen MHC-Molekülen der Klasse I, die auf der Oberfläche einer geeigneten antigenpräsentierenden Zelle exprimiert sind, für eine Zeit, die ausreicht, die genannten CTL auf antigenspezifische Weise zu aktivieren, wobei das Antigen ein Peptid gemäß einem beliebigen der Ansprüche 1 bis 4 ist.

17. Verfahren gemäß Anspruch 16, wobei der CTL und die antigenpräsentierende Zelle allogen bezüglich des MHC-Moleküls der Klasse 1 sind, das das Peptid gemäß einem beliebigen der Ansprüche 1 bis 4 präsentiert.

18. Verfahren gemäß Anspruch 16 oder 17, wobei das Antigen auf die MHC-Moleküle der Klasse 1, die auf der Oberfläche einer geeigneten antigenpräsentierenden Zelle exprimiert werden, geladen werden, indem eine ausreichende Menge des Antigens mit einer antigenpräsentierenden Zelle in Kontakt gebracht wird, wobei die MHC-Moleküle der Klasse 1 der antigenpräsentierenden Zelle vor dem Kontakt im Wesentlichen unbesetzt sind und die MHC-Moieküle der Klasse I nach dem Kontakt im Wesentlichen vollständig besetzt sind.

19. Verfahren gemäß Anspruch 16 oder 17, wobei die antigenpräsentierende Zelle einen Expressionsvektor aufweist, der ein Peptid gemäß Anspruch 7 exprimiert.

20. Verfahren gemäß einem beliebigen der Ansprüche 16 bis 18, wobei das MHC-Molekül der Klasse I HLA-A0201 ist.

21. Aktivierte zytotoxische T-Lymphozyten (CTL), die mittels des Verfahrens gemäß einem beliebigen der Ansprüche 16 bis 20 erhalten werden können und die selektiv eine Zelle erkennen, die ein Polypeptid exprimiert, das eine in Anspruch 1 angegebene Aminosäuresequenz aufweist.

22. Aktivierte zytotoxische T-Lymphozyten (CTL) gemäß Anspruch 21, die selektiv eine maligne Zelle erkennen, die CD45 exprimiert.

23. T-Zell-Rezeptor (TCR), der eine Zelle erkennt, die ein Polypeptid exprimiert, das eine Aminosäuresequenz, wie sie im Anspruch 1 angegeben ist, umfasst, wobei der TCR aus dem zytotoxischen T-Lymphozyten (CTL) nach Anspruch 21 oder 22 erhalten werden kann, oder ein Molekül, das dem TCR funktionell äquivalent ist.

24. T-Zell-Rezeptor (TCR) gemäß Anspruch 23, der eine maligne hämatopoetische Zelle erkennt, die CD45 exprimiert.

25. Polynucleotid, das für einen T-Zell-Rezeptor (TCR) codiert, wie er in den Ansprüchen 23 oder 24 definiert ist.

26. Expressionsvektor, der in der Lage ist, einen T-Zell-Rezeptor (TCR), wie er in den Ansprüchen 23 oder 24 definiert ist, zu exprimieren.

27. Verwendung zytotoxischer T-Lymphozyten, wie sie im Anspruch 21 oder 22 definiert sind, bei der Herstellung eines Medikaments zur Abtötung von Zielzellen in einem Patienten, wobei diese Zielzellen ein Polypeptid exprimieren, das eine im Anspruch 1 angegebene Aminosäuresequenz umfasst.

28. Verwendung zytotoxischer T-Lymphozyten bei der Herstellung eines Medikaments zur Abtötung von Zielzellen in einem Patienten, wobei diese Zielzellen ein Polypeptid exprimieren, das eine im Anspruch 1 angegebene Aminosäuresequenz umfasst, wobei die zytotoxischen T-Lymphozyten mittels eines Verfahrens erzeugt werden, das die Schritte umfasst des (1) Gewinnens zytotoxischer T-Lymphozyten (CTL) aus dem Patienten und des (2) Einbringens eines Polynucleotids in die genannten Zellen, das für einen T-Zell-Rezeptor (TCR) oder ein funktionell äquivalentes Molekül, wie sie in den Ansprüchen 23 oder 24 definiert sind, codiert.

29. Verwendung gemäß Anspruch 27, wobei der Patient einer allogenen Stammzelltransplantation unterzogen wurde.

30. Verwendung gemäß einem beliebigen der Ansprüche 27 bis 29, wobei die Zielzellen Krebszellen sind.

31. Verwendung gemäß Anspruch 30, wobei der Krebs eine Leukämie ist, die das CD45-Polypeptid exprimiert.

32. Verwendung eines aktivierten CTL bei der Herstellung eines Medikaments zur Behandlung eines Patienten mit einer hämatopoetischen malignen Erkrankung, wobei der aktivierte CTL für ein HLA-bindendes Peptid des humanen CD45, wie es im Anspruch 1 definiert ist, spezifisch ist, wobei dieses Peptid von einem HLA-A0201-Molekül im Patienten gebunden ist, und wobei der aktivierte CTL allogen (allorestringiert) bezüglich HLA-A0201 ist, und wobei der Patient eine Stammzelltransplantation von einem Spender erhält, der HLA-A0201-negativ ist, wobei die Stammzelle keine humane embryonale Zelle ist.

33. Verwendung von Stammzellen eines Spenders, der HLA-A0201-negativ ist, bei der Herstellung eines Medikaments zur Behandlung eines Patienten mit einer hämatopoetischen malignen Erkrankung, wobei dem Patienten ein aktivierter CTL verabreicht wird, der für ein HLA-bindendes Peptid des humanen CD45, wie es im Anspruch 1 definiert ist, spezifisch ist, wobei dieses Peptid von einem HLA-A0201-Molekül im Patienten gebunden ist, und wobei der aktivierte CTL allogen (allorestringiert) bezüglich HLA-A0201 ist, wobei die Stammzelle keine humane embryonale Zelle ist.

34. Verwendung gemäß Anspruch 32 oder 33, wobei die aktivierten CTL mittels des Verfahrens nach Anspruch 16 erzeugt werden, wobei die CTL allogen (allorestringiert) bezüglich HLA-A0201 sind.

35. Verwendung gemäß Anspruch 32 oder 33, wobei die aktivierten CTL aus einer Bibliothek von CTL ausgewählt sind.

36. Bibliothek aktivierter CTL, wobei jedes Mitglied der Bibliothek (1) ein Peptid erkennt, wie es im Anspruch 1 definiert ist, wenn es von einem bestimmten, registrierten HLA präsentiert wird, und (2) einen registrierten HLA-Haplotyp aufweist.

37. Bibliothek von Peptiden, wie sie im Anspruch 1 definiert sind, wobei für jedes Mitglied der Bibliothek der Typ des HLA-Moleküls, das es bindet, registriert ist.

## Revendications

1. Peptide constitué de 9 à 12 acides aminés et incluant la séquence d'acides aminés FLYDVIAST ou une variante de cette séquence, cette variante ayant une ou deux substitutions d'acides aminés à l'une et/ou à l'autre des positions 2 et 9 de cette séquence d'acides aminés, et qui est capable de se lier à HLA-A0201.

2. Peptide selon la revendication 1, dans lequel, lorsqu'il est lié à HLA-A0201, le peptide lié à HLA-A0201 est capable de susciter la production d'un lymphocyte T cytotoxique (CTL) qui reconnaît une cellule qui exprime un polypeptide comprenant la séquence d'acides aminés donnée.

3. Peptide selon les revendications 1 ou 2, dans lequel le peptide inclut des liaisons non peptidiques.

4. Peptide selon la revendication 1, consistant en la séquence d'acides aminés FLYDVIAST.

5. Polynucléotide codant un peptide selon l'une quelconque des revendications 1, 2 et 4.

6. Polynucléotide selon la revendication 5, qui et de l'ADN.

7. Vecteur d'expression capable d'exprimer un peptide selon l'une quelconque des revendications 1, 2 et 4.

8. Cellule hôte comprenant un polynucléotide selon la revendication 5 ou 6, ou un vecteur d'expression selon la revendication 7.

9. Procédé de production d'un peptide selon l'une quelconque des revendications 1, 2 et 4, le procédé comprenant la culture de la cellule hôte selon la revendication 8 et l'obtention du peptide à partir de la cellule hôte ou de son milieu de culture.

10. Kit d'éléments comprenant un peptide selon l'une quelconque des revendications 1 à 4 et une cellule présentatrice d'antigène.

11. Kit d'éléments selon la revendication 10, dans lequel la cellule présentatrice d'antigène est une cellule déficiente en l'expression du transporteur de peptide TAP ou n'ayant pas celle-ci.

12. Kit d'éléments selon la revendication 11, dans lequel la cellule est l'une quelconque parmi une cellule T2, une cellule RMA-S ou une cellule de *Drosophila.*

13. Cellule présentatrice d'antigène, dans laquelle ses molécules CMH de classe I sont chargées avec un peptide selon l'une quelconque des revendications 1 à 4.

14. Cellule selon la revendication 13, qui est déficiente en l'expression du transporteur de peptide TAP, ou qui n'a pas celle-ci.

15. Cellule selon la revendication 14, qui est l'une quelconque parmi une cellule T2, une cellule RMA-S ou une cellule de *Drosophila.*

16. Procédé pour produire des lymphocytes T cytotoxiques (CTL) activés, *in vitro,* le procédé comprenant la mise en contact *in vitro* des CTL avec des molécules CMH de classe I humaines chargées d'antigène exprimées sur la surface d'une cellule présentatrice d'antigène appropriée, pendant une période de temps suffisante pour activer, d'une manière spécifique à un antigène, lesdits CTL, dans lequel l'antigène est un peptide selon l'une quelconque des revendications 1 à 4.

17. Procédé selon la revendication 16, dans lequel les CTL et la cellule présentatrice d'antigène sont allogéniques vis-à-vis de la molécule CMH de classe I qui présente le peptide selon l'une quelconque des revendications 1 à 4.

18. Procédé selon la revendication 16 ou 17, dans lequel l'antigène est chargé sur des molécules CMH de classe I exprimées sur la surface d'une cellule présentatrice d'antigène appropriée en mettant en contact une quantité suffisante de l'antigène avec une cellule présentatrice d'antigène, dans lequel, avant le contact, les molécules CMH de classe I de la cellule présentatrice d'antigène sont pratiquement inoccupées et, après le contact, les molécules CMH de classe I sont pratiquement totalement occupées.

19. Procédé selon la revendication 16 ou 17, dans lequel la cellule présentatrice d'antigène comprend un vecteur d'expression qui exprime un peptide selon la revendication 7.

20. Procédé selon l'une quelconque des revendications 16 à 18, dans lequel la molécule CMH de classe I est HLA-A0201.

21. Lymphocytes T cytotoxiques (CTL) activés susceptibles d'être obtenus par le procédé selon l'une quelconque des revendications 16 à 20, qui reconnaissent sélectivement une cellule qui exprime un polypeptide comprenant une séquence d'acides aminés donnée dans la revendication 1.

22. Lymphocytes T cytotoxiques (CTL) activés selon la revendication 21, qui reconnaissent sélectivement une cellule maligne qui exprime CD45.

23. Récepteur de lymphocytes T (TCR) qui reconnaît une cellule qui exprime un polypeptide comprenant une séquence d'acides aminés donnée dans la revendication 1, le TCR étant susceptible d'être obtenu à partir du lymphocyte T cytotoxique (CTL) de la revendication 21 ou 22, ou une molécule fonctionnellement equivalente au TCR.

24. Récepteur de lymphocytes T (TCR) selon la revendication 23, qui reconnaît une cellule hématopoïétique maligne qui exprime CD45.

25. Polynucléotide codant un récepteur de lymphocytes T (TCR) tel que défini dans la revendication 23 ou 24.

26. Vecteur d'expression capable d'exprimer un récepteur de lymphocytes T (TCR) tel que défini dans la revendication 23 ou 24.

27. Utilisation de lymphocytes T cytotoxiques tels que définis dans la revendication 21 ou 22, pour la préparation d'un médicament destiné à tuer des cellules cibles chez un patient, ces cellules cibles exprimant un polypeptide comprenant une séquence d'acides aminés donnée dans la revendication 1.

28. Utilisation de lymphocytes T cytotoxiques pour la préparation d'un médicament destiné à tuer des cellules cibles chez un patient, ces cellules cibles exprimant un polypeptide comprenant une séquence d'acides aminés donnée dans la revendication 1, dans laquelle les lymphocytes T cytotoxiques sont produits par un procédé comprenant les étapes consistant (1) à obtenir des lymphocytes T cytotoxiques (CTL) auprès du patient ; et (2) à introduire dans lesdites cellules un polynucléotide codant un récepteur de lymphocytes T (TCR), ou une molécule fonctionnellement équivalente, tel que défini dans les revendications 23 ou 24.

29. Utilisation selon la revendication 27, dans laquelle le patient a subi une transplantation de cellules souches allogéniques.

30. Utilisation selon l'une quelconque des revendications 27 à 29, dans laquelle les cellules cibles sont des cellules cancéreuses.

31. Utilisation selon la revendication 30, dans laquelle le cancer est une leucémie qui exprime le polypeptide CD45.

32. Utilisation d'un CTL activé pour la préparation d'un médicament destiné à traiter un patient ayant une malignité hématopoïétique, dans laquelle le CTL activé est spécifique pour un peptide liant le HLA de CD45 humain tel que défini dans la revendication 1, ce peptide étant lié par une molécule HLA-A0201 chez le patient, et ce CTL activé étant allogénique (allorestreint) vis-à-vis de HLA-A0201, et dans laquelle le patient reçoit une transplantation de cellules souches d'un donneur qui est HLA-A0201 négatif, dans laquelle la cellule souche n'est pas une cellule embryonnaire humaine.

33. Utilisation de cellules souches d'un donneur qui est HLA-A0201 négatif pour la préparation d'un médicament destiné à traiter un patient ayant une malignité hématopoïétique, dans laquelle le patient reçoit un CTL activé qui est spécifique pour un peptide liant le HLA de CD45 humain tel que défini dans la revendication 1, ce peptide étant lié par une molécule HLA-A0201 chez le patient, et dans laquelle le CTL activé est allogénique (allorestreint) vis-à-vis de HLA-A0201, dans laquelle la cellule souche n'est pas une cellule embryonnaire humaine.

34. Utilisation selon la revendication 32 ou 33, dans laquelle les CTL activés sont fabriqués par le procédé de la revendication 16 dans lequel les CTL sont allogéniques (allorestreints) vis-à-vis de HLA-A0201.

35. Utilisation selon la revendication 32 ou 33, dans laquelle les CTL activés sont choisis dans une banque de CTL.

36. Banque de CTL activés dans laquelle chaque membre de la banque (1) reconnaît un peptide tel que défini dans la revendication 1 lorsqu'il est présenté par un HLA enregistré particulier et (2) a son haplotype HLA enregistré.

37. Banque de peptides tels que définis par la revendication 1, dans laquelle, pour chaque membre de la banque, la sorte de molécule HLA qu'il lie est enregistrée.


### SEQUENCE LISTING

<110> Imperial College Innovations
<120> Immunotherapeutic Methods and Molecules
<130> ICOW/P23777PC
<140>
   <141>
<160> 16
<170> PatentIn Ver. 2.0
<210> 1
   <211> 9
   <212> PRT
   <213> Homo sapiens
<400> 1
   Phe Leu Tyr Asp Val Ile Ala Ser Thr
    1               5
<210> 2
   <211> 9
   <212> PRT
   <213> Homo sapiens
<400> 2
   Ala Leu Ile Ala Phe Leu Ala Phe Leu
   1 5
<210> 3
   <211> 9
   <212> PRT
   <213> Homo sapiens
<400> 3
   Lys Leu Phe Thr Ala Lys Leu Asn Val
   1 5
<210> 4
   <211> 9
   <212> PRT
   <213> Homo sapiens
<400> 4
   Met Ile Trp Glu Gln Lys Ala Thr Val
   1 5
<210> 5
   <211> 9
   <212> PRT
   <213> Homo sapiens
<400> 5
   Asn Leu Ser Glu Leu His Pro Tyr Leu
   1 5
<210> 6
   <211> 10
   <212> PRT
   <213> Homo sapiens
<400> 6
   Val Asn Leu Ser Glu Leu His Pro Tyr Leu
   1 5 10
<210> 7
   <211> 9
   <212> PRT
   <213> Homo sapiens
<400> 7
   Leu Leu Ala Phe Gly Phe Ala Phe Leu
   1 5
<210> 8
   <211> 9
   <212> PRT
   <213> Homo sapiens
<400> 8
   Tyr Leu Tyr Asn Lys Glu Thr Lys Leu
   1 5
<210> 9
   <211> 9
   <212> PRT
   <213> Homo sapiens
<400> 9
   Leu Ile Leu Asp Val Pro Pro Gly Val
   1 5
<210> 10
   <211> 10
   <212> PRT
   <213> Homo sapiens
<400> 10
   Thr Leu Ile Leu Asp Val Pro Pro Gly Val
   1 5 10
<210> 11
   <211> 9
   <212> PRT
   <213> Homo sapiens
<400> 11
   Ile Leu Tyr Asn Asn His Lys Phe Thr
   1 5
<210> 12
   <211> 9
   <212> PRT
   <213> Homo sapiens
<400> 12
   Ile Leu Pro Tyr Asp Tyr Asn Arg Val
   1 5
<210> 13
   <211> 9
   <212> PRT
   <213> Homo sapiens
<400> 13
   Tyr Ile Leu Ile His Gln Ala Leu Val
   1 5
<210> 14
   <211> 9
   <212> PRT
   <213> Homo sapiens
<400> 14
   Phe Gln Leu His Asp Cys Thr Gln Val
   1 5
<210>15
   <211> 10
   <212> PRT
   <213> Homo sapiens
<400> 15
   Lys Leu Leu Ala Phe Gly Phe Ala Phe Leu
   1 5 10
<210> 16
   <211> 10
   <212> PRT
   <213> Homo sapiens
<400> 16
   Tyr Gln Tyr Gln Tyr Thr Asn Trp Ser Val
    1              5                   10